# EUROPEAN PATENT APPLICATION

(11) **EP 4 728 865 A1**
(43) Date of publication of application: **22.04.2026**
(21) Application number: 24206733.8
(22) Date of filing: 15.10.2024
(51) Int. Cl.: A01N 37/46, A01K 51/00, A01N 63/10, A01N 63/50, A01N 65/00

(54) **METHOD FOR COMBATING PESTS ON BEES AND IN THE BEEHIVE**

(71) Applicant: Bergmann, Sven, 17498 Gristow (DE)
(72) Inventor: BERGMANN, Sven, 17498 Gristow (DE)
(74) Representative: Gille Hrabal Partnerschaftsgesellschaft mbB Patentanwälte

(57) **Abstract**

The present invention relates to a method for combating animal pest infections and/or infestations on bees and in the beehive by applying an animal pest marking agent and thereby marking the animal pest in such a way that they are detected and successfully combated by the bees in a defensive response. Particularly, the method relates to combating mite infestations, like infestations with the Varroa mite, the mite *Acarapis woodi,* or other mites or infestations with roundworms (nematodes). The invention further relates to a method for the production of the animal pest marking agent and the use thereof for the prophylactic and therapeutic treatment of bees against animal pest infestations, like infestation with the Varroa mite.

## Description

### INTRODUCTION

The present invention relates to a method for combating animal pest infections on bees and in the beehive by applying an animal pest marking agent and thereby marking the animal pest in such a way that they are detected and successfully combated by the bees in a defensive response. Particularly, the method relates to combating mite infestations, like infestations with the Varroa mite or the mite *Acarapis woodi* or infestations with roundworms (nematodes). The invention further relates to a method for the production of the animal pest marking agent and the use thereof for the external prophylactic and therapeutic treatment of bees against animal pest infestations, like infestation with the Varroa mite.

### BACKGROUND OF THE INVENTION AND STATE OF THE ART

Pest infestations can cause massive damages to domesticated and/or farmed western bees. In particular, infestations with mites like the Varroa mite or the mite *Acarapis woodi* or infestations with roundworms (nematodes) cause particular problems in beekeeping.

Numerous attempts to render pests, like mites or roundworms (nematodes), particularly the Varroa mite, harmless or to combat them in the long term without using substances that are harmful to human health have so far been more or less unsuccessful. In 2013, the Varroa mite, which infested bee colonies, caused damage amounting to US$ 300 billion worldwide.

According to current knowledge, mites like the Varroa mite are combated in bees by applying combating compounds like organic acids, essential oils or approved pesticides (biocides). However, organic acids, essential oils and approved pesticides have the disadvantage of leaving residual detectable traces of the applied compounds on the bees as well as on the bees' products (like honey or wax) and in the environment. Further, since mites, like the Varroa mite, are insects like the bee, the application of known combating compounds often results in damage to the bees themselves, their products and the environment.

Numerous mixtures or biocides are already known for controlling pests such as the Varroa mite or the mite *Acarapis woodi* (USA) which, however, also attack honeybees and their larvae. Some of these substances are not approved in several countries and some are prescription drugs and can only be administered by veterinary personnels.

It is further well known to combat Varroa mites by treating the beehives with various organic acids, including oxalic acid, formic acid, lactic acid, which are often applied in very high concentrations, for example 60% formic acid.

Essential oils are also used *[*Imdorf A. und P. Blumer-Meyere (2002) Lässt sich Varroa destructor mit ätherischen Ölen bekämpfen? Schweizer Zentrum für Bienenforschung, Bienenzeitung 1-14*],* including eucalyptus oil, menthol or camphor. Applying essential oils onto the beehive requires the use of a variety of equipment and often leads to the nebulization or evaporation of these substances. Some vegetable oils, like bitter melon oil (80%), thyme oil (76%), thymol and garlic oil (72%) are also used to combat the Varroa mite. These substances are often subject to strict application criteria, as they can also harm the bees and/or their brood or even destroy them in the event of accidents or improper use. The honey obtained from accordingly treated beehives has also been proven to contain at least traces, but also higher concentrations, of residual combating compounds. When using essential oils as a combating agent, the residual contents thereof may lead to a deterioration of the quality of the bees' products, for example, the honey can smell or taste of menthol or garlic after the beehives have been treated with thymol or garlic oil.

Further known methods for combating mites on bees and in the beehives comprise the heating of the beehive or individual parts thereof to enhanced temperatures, typically by subjecting the beehives to a heat treatment at up to 37 - 41 °C. For such heat treatment specific equipment is used like specific honeycomb, floor or wall heating systems. This method is often referred to as a bee sauna. However, applying such heat treatment requires the necessity of specific cost intensive heating systems and therewith high upfront costs and additional time effort for the beekeeper.

Other approaches are being taken with the selective breeding of Varroa mite-resistant bees. On the one hand, bee colonies can be bred that defend themselves against specific pests like Varroa mite infestation far more actively than others through an increased grooming instinct. On the other hand, queens can be mated that have already demonstrated a certain resistance in their offspring. In both cases, the worker bees recognize infected larvae and remove them from the honeycombs, which is interpreted as hygienic behavior on the part of the bees [Oddie M., Büchler R., Dahle B., Kovacic K., Le Conte Y., Locke B., de Miranda J.R., Mended F. and Neumann P. (2018) Rapid parallel evolution overcomes global honey bee parasite. Sci Rep 8, 7704*].*

Cutting out the drone brood in summer is helpful, also in combination with one or more of the measures mentioned, as a well-known step by beekeepers to prevent or reduce Varroa mites.

Despite all the disadvantages, some beekeepers prefer pesticides or miticides such as Tau-Fluvalinate or Coumaphos, which are available on the market in various forms. However, there is always the risk of damage or complete destruction of the bees as well as the risk of the development of resistances. Detectable residual traces of these substances in honey is not uncommon.

Further, with all of the control measures available so far and discussed above, success can be determined only after 3 to 5 days based on the number of mites in the hive. Therewith, a further disadvantage of the available methods is the requirement of long-time breeding efforts and that a quick solution against occurring infestations cannot be achieved. Further, even previously highly effective bio- or insecticides bear the risk and have been found to be losing their effectiveness over the years due to the development of resistance in the target pests.

Regarding the above-described disadvantages, there is therefore an ongoing need to provide effective and safe methods for treating bees, particularly honeybees, against pests like mites or roundworms. It is particularly important to provide methods which avoid the disadvantages of the so far available methods and allow effective treatment in a way that no harmful or deteriorating residual combating agents remain. It is particularly preferred that no biocides are used due to the above-described disadvantages deriving from their use.

### OBJECT OF THE INVENTION

The object of the invention is to provide a method for effectively combating parasitic infestations in bees and beehives. Particularly, such method should allow the use of pesticides or biocides to be avoided. The method must not deteriorate, damage or harm the bees and the beehives as well as the bees' produce like honey, beeswax, flower pollen, bee bread, propolis or royal jelly. A particular object of the invention can be seen in providing new substances and methods for combating mites and roundworms on bees and in the beehive, specifically for combating the Varroa mite. The novel substances and methods should allow a highly effective and easy to apply treatment. It should provide quick and long-lasting results and should further be well-tolerable and safe to the bees and the products derived from the bees and the beehive. The method should furthermore provide a quick solution to an occurring infestation as well as the suitability for prophylactic treatment to avoid occurrence and to contain the spread. A particular object of the invention can therefore be seen in providing a method, application form and agents allowing the problem-free use of biological and/or abiotic agents that do not have a negative impact on the well-being of the bees and the beehive and the produce obtained therefrom. In particular, the novel method, application form and agents should be safe without causing mortality in the bees and should be highly effective in all age groups within 24 hours up to 7 days. No residues should be detectable and the method and substances to be applied must be safe for the bee products and human health.

### DESCRIPTION OF THE INVENTION

The inventors of the present invention developed a novel method for combating pests, like mites and roundworms, on bees and in beehives, which neither harms nor affects the bees, their produce or the environment. The novel method is based on a method of marking the pests to be combated, like the mites, in such a way that they can be detected and successfully combated by the bees in all age groups and in all locations, except in the capped honeycomb. In the method of the invention the pests, e.g. the mites like Varroa mites, are marked or labelled by a biotic or abiotic agent being able to effectively mark the pest so that the host, like the bees, are able to identify and combat the marked pest in a self-defense reaction mechanism. As an effective example of such a pest marking agent antibodies can be mentioned, which can be produced in animals (including mammals and fish) after immunization with the target pests in the form of the whole pests or their components, followed by harvesting antibody-enriched fluids (like blood or serum) and isolating these antibody-enriched fluids from the immunized specimen (animals; also designated as "donor specimen" or "donor animals"). In the method of the invention such antibody-enriched pest marking agent can then be applied on the bees and in the beehive.

The inventors of the present invention surprisingly found that with the method of combating pests by applying a pest marking or pest labelling agent, like an antibody-enriched pest marking agent, it is possible to effectively and selectively mark the target pest for a defensive response of the host animal, namely the bees. The biotic or abiotic pest marking agent according to the invention, like the antibody-enriched pest marking agent, may be provided in a form dissolved in water and can thus easily be applied to infested bees or their housing. The inventors surprisingly found that the application of such a pest marking agent can effectively control the pests without harming the bees and without leaving undesired deteriorating or harmful residues in the bees' produce. It was furthermore found that the desired combating effects occur quickly after the application, i.e. after only up to one day.

In the sense of the present invention "combating" and/or "controlling" comprises a reduction of the number of pests and/or their development stages (e.g. nymphs in any stadium) and the prevention of the reproduction, development and hatching of pests.

With the method of the present invention, it becomes possible to selectively mark the target pest, which ensures that only the target pest, e.g. the mite is marked in the hive. Other animals such as the bees themselves or beetles and spiders tolerated in the hive are not affected or marked. The inventors made the surprising observations that within an hour after applying a pest marking agent comprising harvested and concentrated / isolated antibodies of the target pest in a dissolution with water, by spraying onto the honeycomb or into the honeycomb lane, the pest like mites flee en masse both from the bees directly and from the hive itself. Even more surprising it has been observed that this effect lasts up to seven days.

Since this method of marking the pests, like mites, does not harm or affect the bees, their produce (for example honey, beeswax, flower pollen, bee bread, propolis or royal jelly) or the environment at all, this new treatment method can be carried out at any time of the year and as often as necessary, not only at one or two specific times of the year like required for other conventional methods.

Further, the application of the pest marking agent, like antibody-enriched pest marking agent, can be done by simple fogging or spraying with conventional beekeeping equipment and can be done by the beekeeper during routine checks of the bees and honeycombs or when the beekeeper routinely removes the honeycombs from the frames. The treatment method of the invention can thus easily be implemented in the routine beekeeping and no particular time-consuming extra effort is necessary.

It has been observed that the light spray or fog of an antibody-enriched pest marking agent does not disturb the bees. It has been observed that marking free mites with the method of the invention allows to reduce their number in the hive and on the bees to almost 99% within 1 to 24 hours, with the effect lasting up to seven days compared to hives treated only with water or with mite-negative sera of the same pest species. It was further found that the novel treatment method allows to combat the mites on newly hatched bees, which results in a stop of a development of a new generation of mites.

It is possible, to combine the novel method of the invention with conventional treatments with acids, pesticides or essential oils, if necessary or wished, although it is preferred to avoid using acids, pesticides or essential oils by replacing such conventional treatments by the novel method of the present invention.

The present invention offers significant advantages and improvements in the keeping of bees and the control of pests in the beehive, as it offers safe, effective, simple to apply, quick and long-lasting options of pest combat and no longer requires any waiting time for honey and other bees' products.

The new method further offers a wide variety and flexibility in providing the antibody-enriched pest marking agent to be applied.

Accordingly, a first aspect of the invention relates to a method for combating animal pest infections on bees and in the beehive by applying a biotic or abiotic pest marking agent being able to effectively mark the target pest so that the host, like the bees, are able to identify and combat the marked pest in a self-defense reaction mechanism, such as preferably an animal pest marking agent comprising an effective amount of antibodies of the target animal pest to be combated, onto the bees and/or the beehive.

Therein, in a preferred aspect, the animal pest marking agent can be considered as an antibody-enriched agent comprising antibodies of the target animal pest to be combated and the enrichment with such antibodies takes place in a degree that an effective amount of the antibodies is comprised therein, allowing to mark the target pest when applied thereon, e.g. on the bees and/or in the beehive. The marking of the pests with the antibodies has the effect that the so-marked pests are detected and combated by the bees in a defensive response. Concretely, the antibody-marking or -labelling of the pests, like mites, triggers a defensive reaction in the bee colony with the effect that the bees attack the pest (e.g. the mites) so that they drive the pests out of the hive.

The target animal pests can be selected from any animal pest, which can be detected and attacked by the bees in a defensive reaction upon labelling with the pest marking agent, like antibody-marking. Preferably the animal pests are selected from mites and roundworms (nematodes).

Target animal pests from the group of mites can be selected from the Varroa mite (including Varroa destructor and/or Varroa jacobsoni) and *Acarapis woodi.*

Preferably, the target animal pests are selected from mites, preferably from the Varroa mite and *Acarapis woodi,* more preferably the target animal pest is the Varroa mite.

For practical reasons the animal pest marking agent can be provided in frozen or freeze-dried form, e.g. in the form of a frozen or freeze-dried antibody-enriched fluid or concentrate, which allows stable storage and easy distribution thereof.

Regarding such frozen embodiments, it is advantageous to store the animal pest marking agent, e.g. as an antibody-enriched concentrate, in frozen form at about -20°C until use. Alternative and further preferred options for storage, handling and shipping comprise freeze-dried forms.

However, for applying the animal pest marking agent onto the bees and/or beehives, it is preferably provided in or transferred into the form of an aqueous solution, i.e. in sprayable form. Such aqueous solutions can, for example, be obtained by dissolving or diluting the frozen or freeze-dried antibody-enriched concentrate or by using the harvested and isolated concentrate directly, optionally after further dissolution to obtain the desired concentration. Preferably, frozen or freeze-dried antibody-enriched concentrates of the animal pest marking agent are used, dissolved or diluted with water in a ratio of 1 : 100 to 1 : 16,000, preferably of 1 : 200 to 1 : 16,000 or higher. Preferably such solution is prepared shortly prior to use in the method according to the invention, i.e. before spraying onto the bees and/or beehive. The suitable dissolution ratio can be determined depending on the antibody titer against the pest obtained from the donor specimen.

Such aqueous solutions of pest marking agents can contain further harmless excipients, like diluents being acceptable to the bees, the beehives and the bees' produce. In principle, it is also possible to add conventional treatment agents like acids, pesticides or essential oils, however, for the reasons discussed above this is less preferred.

In the method according to the invention the animal pest marking agent is preferably applied onto the bees and/or the beehive by spraying, nebulizing or fogging an aqueous solution or dilution of the antibody-enriched animal pest marking agent. The solution can be applied either by placing it above the honeycombs in the hive or, preferably, in the form of fine droplets by targeted nebulization into the hive. The bees do not react aggressively in any way to the light spraying or fogging with the animal pest marking agent contained in the water.

The animal pest marking agent according to the invention can be sprayed and/or nebulized into the bee colony, preferably on each side of a honeycomb and/or frame during a routine inspection of the colony, but also directly into the honeycomb lane. The nebulizer or sprayer is usually guided in a meandering motion so that as many animals in the frame as possible are wetted with the solution.

The pest marking agent can be applied by direct misting onto the bees in the frames and/or honeycombs, or directly between the frames of a frame at any time of the year for interval control of mites according to their reproduction cycle, or for prevention at any time. Treatment with the solution does not result in any waiting times for the bees, wax or honey. The components of the solution cannot be detected in beekeeping products, as they are only found on the pests, like mites.

Applying the pest marking agent in the form of a solution has the advantageous property that the pest's surface is wetted without the pest being able to avoid it. The solution covers and marks the pests, like mites, on the outer cuticle. It does not penetrate the pest but remains on the outer cuticle. The solution has no effect on other living creatures such as bees. However, double marking, bee and pest, to increase the bees' grooming instinct is possible.

Preferably, the pest marking agent of the invention comprises an antibody-enriched component diluted in drinking water or other harmless diluents which have good wetting properties and are tolerable to the bees. Preferably, no further diluents or solvents (besides water) are added.

It was found that a solution in water can effectively control the pests by marking or labelling the parasites (pests) without harming the bees. The effect of labelling the pests upon application of the antibody-enriched animal pest marking agent of the invention occurs due to the fact that the antibody-enriched animal pest marking agent comprises specific antibodies of the target pests only. Therefore, it does not harm other species like the bees. The pest marking agent directly marks the free pests, like mites, in the hive as well as those on and around the bees themselves.

Regarding the effective amounts of the target pest specific antibodies in the pest marking agent it is necessary that the antibody-concentrations are sufficiently high to effect marking of the pests to be combated. Depending on the degree of infestation, the concentration of the agent can be adjusted as required without becoming harmful to the bees. That means, even if higher concentrations are required because of a massive infestation this is uncritical with respect to the bees, the beehives and the bees' produce. In fact, it has been found that no restrictions regarding an upper limit were found, that means, even at high concentrations no detrimental effects on the bees were observed. That means, the concentration of the antibody-enriched pest marking agent is only significant if it is too low, i.e. too diluted, which may lead to no or no sufficient marking of all pests.

Due to the high tolerability and safety of the pest marking agent in the method of the invention the application of the animal pest marking agent can be repeated one or more times, e.g. until all pests are combated. It is further possible to apply the animal pest marking agent prophylactically to avoid infection or outbreaks of pest infestation.

In principle, it is possible to apply the herein described method of combating pests also to other animals being affected by pest infestation and which have a similar self-defense reaction mechanism on the pests which can be triggered by the antibody-marking as described herein.

A further aspect of the invention relates to a method for the preparation of an antibody-enriched animal pest marking agent which comprises the steps of
(a) infecting and/or exposing a specimen (donor specimen) with the target animal pest or biological residues of the target animal pest,
(b) harvesting antibody-enriched fluids (like blood or serum) from the immunized donor specimen, and
(c) optionally concentrating the harvested antibody-enriched fluids wherein the antibody-enriched fluids and/or concentrate comprises antibodies against the target animal pest capable of marking the target animal pest for a defensive response of the infested animals (infested host), like bees.

The method can further comprise a step of
(d) freezing or freeze-drying the isolated antibody-enriched fluids and/or concentrate of step (b) or (c) and/or
(e) diluting the isolated antibody-enriched fluids and/or concentrate of step (b) or (c) or the frozen or freeze-dried isolated antibody-enriched fluids and/or concentrate of step (d) in water or in an aqueous solution to provide the antibody-enriched animal pest marking agent in the form of an aqueous solution.

In accordance with the explanations provided above, in the preparation method the solution or dilution of the antibody-enriched fluids and/or concentrate in water can be prepared in a ratio of antibody-enriched fluids / concentrate:water of between 1 : 100, preferably 1 : 200 and 1 to 16000 or higher.

Suitable specimen (donor specimen) which can be exposed to the target animal pest or biological residues comprise vertebrates and invertebrates. Examples of vertebrates comprise mammals, birds, reptiles, amphibians and fish. Preferred vertebrates are fish and among them more preferably the vertebrates are selected from the families *Cyprinidae Percidae, Acipenseridae, Salmonidae, Arapaimidae* or from the order *Siluriformes.*

Preferably the donor specimen to be exposed to the target animal pest or biological residues is selected from fish, reptiles and amphibians.

In the method according to the invention the antibody-enriched fluids and/or concentrate is preferably obtained from sera by collecting blood from the immunized donor specimen having been exposed to the target animal pest or biological residues thereof, allowing the blood to clot, and separating serum and solid residues by centrifugation and / or filtration. The solid residues are usually discarded while the sera containing the target antibodies are collected.

Suitable biological residues of the target animal pest comprise the cuticula and / or the entire target animal pest.

Preferably, the target pests are selected from mites and roundworms (nematodes), preferably the target pests are selected from mites, like the Varroa mite or *Acarapis woodi,* more preferably the target pest is the Varroa mite.

Besides the herein described method of deriving the target antibodies naturally occurring in the serum after immunization of (donor) animals, further possibilities of cultivating, harvesting and/or isolating the target antibodies or similarly suitable pest marking agents exist, including artificial, molecular biological, genetic engineering and/or protein chemical production of the pest labelling agents, like the target antibodies or their proteins. Accordingly, it is also possible to gain pest labelling agents, like the target antibodies for animal pest marking agents for the use in the method for combating pests as described herein by artificial, molecular biological, genetic engineering and/or protein chemical production of the pest labelling agents, like the target antibodies or their proteins. It is further possible to provide pest marking agents specifically tailored to a pest by using antibodies, which can be monoclonal, polyclonal or monospecific, from different animals against corresponding pests. However, it is important that the marking of the pest triggers the recognition and defense reaction of the infected host and that the marked pest recognizes this and escapes.

The invention further relates to antibody-enriched animal pest marking agents being suitable for marking selected target pests therefore being suitable in the use of combating the selected pests specifically via defense reaction of the infected host.

In a further aspect the invention relates to novel pest marking agents comprising a biotic or abiotic agent being able to effectively mark the target pest so that the host, like the bees, are able to identify and combat the marked pest in a self-defense reaction mechanism. Particularly, such novel pest marking agents comprise antibody-enriched animal pest marking agent comprising antibodies against the target pest, preferably against mites, more preferably against Varroa mites.

In a further aspect the invention relates to antibody-enriched animal pest marking agents obtainable by the methods as described herein.

Accordingly, the present invention further relates to a medicament comprising the biotic or abiotic agents being able to effectively mark the pest so that the host, like the bees, are able to identify and combat the marked pest in a self-defense reaction mechanism, like preferably the antibody-enriched animal pest marking agents described herein.

The invention further relates to a pest marking agent comprising an effective amount of a biotic or abiotic agent being able to effectively mark the target pest so that the host, like the bees, are able to identify and combat the marked pest in a self-defense reaction mechanism, for the use for prophylaxis and treatment of diseases related to pest infestation in bee colonies, preferably for the use in the prophylaxis and treatment of varroosis in bee colonies caused by the Varroa mite.

The invention further relates to an antibody-enriched animal pest marking agent comprising an effective amount of antibodies against a target animal pest, such as preferably an antibody-enriched animal pest marking agent obtainable by the method as described herein, for the use in the prophylaxis and/or treatment of diseases related to pest infestation in bee colonies, preferably for the use in the prophylaxis and treatment of varroosis in bee colonies caused by Varroa mites and other mites.

The pest marking agent and its use in the method of the present invention turned out to be extremely advantageous for treating honeybees and to combat mites, in particular Varroa mites. With the method of the invention, comprising the application of the pest marking agent as described herein, a reduction in mite infestation of more than 95% compared to non-treated reference colonies after an application period of 1 to 24 hours up to 7 days has been achieved as shown in more detail in the Examples. No noticeable damage to the active bee colony or the brood was observed. It was also found that the pest marking agent can be applied preventively, and as a result mite infestation, including renewed infestation, is largely avoided and/or significantly reduced.

The invention is further illustrated, without being limited thereto, by the following Examples.

### EXAMPLES

### I. Preparation of an Antibody-Enriched Varroa Mite-Marking Solution

An antibody-enriched solution for marking Varroa mites was prepared by contacting a donor animal with Varroa mites for immunization, followed by blood collection from the immunized animal. The blood is allowed to clot at a temperature between 10°C for 4 h or 22°C for 15 to 30 min. After direct decantation of the serum from the blood clot or after centrifugation at low speed for separating blood clot from serum and decanting afterwards, the obtained sera are stored at least at -20°C.

### II. Evaluation of the Effect of the Varroa Mite labelling Solution in Combating Varroa Mites on Bees and in the Beehives

The effect of the antibody-enriched Varroa mite-marking solution according to the invention, obtained according to Example I, was investigated in the treatment of honeybees with Varroa mite infestation.

For the test arrangement, three bee colonies (R, L and S) were selected that were also important for honey production, but for which no mite prophylaxis was carried out previously. The colony strength was approximately 35 - 40,000 bees per hive. The colonies are housed in so-called magazine hives with three frames each with 11 honeycombs and were examined for their stocking, colony strength, brood and general condition and found to be good.

Three days before the start of the treatments, a diaper coated with sunflower oil was placed in each hive (3 frames with 11 honeycombs each) to determine the number of mites in the hive. After three days, the number of mites was counted on the diaper using a magnifying glass.

The following mite counts were conducted on the untreated hives as the starting point:

| | **Hive S** | **Hive R** | **Hive L** |
|---|---|---|---|
| **No. of mites on oiled diaper** | 128 | 77 | 31 |
| **No. of mites on 40 bees** | 13 | 7 | 8 |

Hive S was carefully misted with the antibody-enriched solution of Example I, comprising positive serum against Varroa mites. For comparison, hive R was misted with a negative serum, obtained in the same way from a donor specimen which never had any contact to Varroa mites but belongs to the same species from which the positive serum was obtained and hive L was misted with simple drinking water as an additional negative control.

To spray the solutions and/or water onto the individual honeycombs, a commercially available sprayer with a piston was screwed onto a filled 500 ml plastic bottle to increase the pressure. After the solution was poured into the bottle, which was inverted at least 10 times to evenly distribute the substance, excess pressure was generated using the sprayer's hand piston and each individual frame of the hive was carefully sprayed on both sides in a meandering pattern for approx. 3 - 5 seconds.

The following mite counts were conducted after treating the hives with the test and control solutions by counting the number of mites that had fallen on the oiled diapers after 1, 2, 5 and 24 hours using a magnifying glass:

| **No. of mites fallen on the oiled diapers** | **Hive S (positive serum)** | **Hive R (negative serum)** | **Hive L (water)** |
|---|---|---|---|
| **After 1 h** | 13 | 2 | 4 |
| **After 2 h** | 11 | 3 | 4 |
| **After 5 h** | 21 | 4 | 5 |
| **After 24 h** | 45 | 4 | 11 |

Apart from the mites, no other animals (bees, tolerated beetles or spiders) in the hives were affected in any way. However, in hive S the mites showed an active movement to escape from the hive.

After one hour of treatment, only juvenile nymphs were detected in the three hives. At the evaluation timepoints the following mite and nymph counts were conducted:

| **No. and stage of nymphs on the diapers** | **Hive S (positive serum)** | **Hive R (negative serum)** | **Hive L (water)** |
|---|---|---|---|
| **After 1 hour - mites** | 13 | 2 | 4 |
| **After 2 hours - adults** | 7 | 3 | 0 |
| **After 2 hours - nymph stages** | 4 | 1 | 3 |
| **After 5 hours - adults** | 16 | 5 | 3 |
| **After 5 hours - nymph stages** | 5 | 0 | 1 |
| **After 24 hours - adults** | 24 | 8 | 1 |
| **After 24 hours - juvenile mites** | 21 | 0 | 0 |
| **After 24 hours - nymph stages** | 0 | 3 | 3 |
| **After 24 hours - mites on 40 bees** | 0* | 7 | 5 |

| | | | |
|---|---|---|---|
| * one bee had a mite on its wing and it was moving away from the bee | | | |

The results induced 100% effectiveness 24 hours after application without affecting the bee colonies in hives S, R and L in any way. No mites were found attached or stuck on the 40 bees in hive S 24 hours after treatment. One bee had a mite on its wing and it was moving away from the bee. On the 40 bees in hives L and R, 5 and 7 mites were found on the bees respectively.

## Claims

1. A method for combating animal pest infections on bees and in the beehive by applying an animal pest marking agent comprising an effective amount of a biotic or abiotic agent being able to effectively mark the target animal pest so that the host, like the bees, identify and combat the marked pest in a self-defense reaction mechanism.

2. The method according to claim 1, wherein the animal pest marking agent is an antibody-enriched agent comprising antibodies against the target animal pest to be combated in an effective amount allowing to mark the target pest on the bees and/or in the beehive with the antibodies so that the pests are detected and combated by the bees in a defensive response and effectively reduced.

3. The method according to claim 1 or 2, wherein the target pests are selected from mites and roundworms (nematodes),
preferably the target pests are selected from mites, including the Varroa mites and the mite *Acarapis woodi,*
more preferably the target pest is the Varroa mite.

4. The method according to any one of claims 1 to 3, wherein the animal pest marking agent is present
• in the form of an aqueous solution, or
• in the form of an aqueous dilution of a frozen or freeze-dried pest marking agent.

5. The method according to any one of claims 1 to 4, wherein the animal pest marking agent is applied on the bees and in the beehive by spraying, nebulizing or fogging an aqueous solution and/or dilution of the pest marking agent.

6. The method according to any one of claims 1 to 5, wherein the application of the animal pest marking agent is repeated one or more times.

7. A method for the preparation of an antibody-enriched animal pest marking agent comprising the steps of
(a) infecting and/or exposing a donor specimen with the target animal pest or biological residues of the target animal pest,
(b) harvesting antibody-enriched fluids from the donor specimen, and
(c) optionally concentrating the harvested antibody-enriched fluids,
wherein the antibody-enriched fluids and/or concentrate comprises antibodies against the target animal pest capable of marking the target animal pest for a defensive response of bees.

8. The method according to claim 7 further comprising a step of
(d) freezing or freeze-drying the isolated antibody-enriched concentrate of step (c) and/or
(e) diluting the isolated antibody-enriched concentrate of step (c) or the frozen or freeze-dried isolated antibody-enriched concentrate of step (d) in water or in an aqueous solution to provide the antibody-enriched animal pest marking agent in the form of an aqueous solution.

9. The method according to any one of claims 7 to 8, wherein the donor specimen is selected from fish, reptiles and amphibia being exposed to the target animal pest or biological residues thereof,
preferably the donor specimen is selected from vertebrates, such as selected from fish,
more preferably the donor specimen are selected from the families *Cyprinidae Percidae, Acipenseridae, Salmonidae, Arapaimidae* or from the order *Siluriformes.*

10. The method according to any one of claims 7 to 9, wherein the antibody-enriched fluid is blood serum obtained by collecting blood from the immunized donor specimen being exposed to the target animal pest or biological residues thereof, allowing the blood to clot, and separating serum and solid residues by discarding, centrifugation and/or filtration.

11. The method according to any one of claims 7 to 10, wherein the target pests are selected from mites and roundworms (nematodes), preferably the target pests are selected from mites, including the Varroa mite and, the mite *Acarapis woodi,* more preferably the target pest is the Varroa mite.

12. A pest marking agent comprising a biotic or abiotic agent being able to effectively mark a target pest so that the host is able to identify and combat the marked pest in a self-defense reaction mechanism.

13. The pest marking agent according to claim 12, which is an antibody-enriched animal pest marking agent comprising antibodies against the target pest, preferably against mites, more preferably against Varroa mites.

14. A pest marking agent comprising an effective amount of a biotic or abiotic agent being able to effectively mark the target pest so that the host, like the bees, are able to identify and combat the marked pest in a self-defense reaction mechanism, for the use for prophylaxis and treatment of diseases related to pest infestation in bee colonies, preferably for the use in the prophylaxis and treatment of varroosis in bee colonies caused by the Varroa mite.

15. The pest marking agent for the use according to claim 14 wherein the pest marking agent is an antibody-enriched animal pest marking agent.
